# EUROPÄISCHE PATENTSCHRIFT

(11) **EP 1 313 510 B1**
(45) Veröffentlichungstag und Bekanntmachung des Hinweises auf die Patenterteilung: **13.10.2004**
(21) Anmeldenummer: 01983443.1
(22) Anmeldetag: 21.08.2001
(51) Int. Cl.: A61K 47/14

(54) **INJIZIERBARE PHARMAZEUTISCHE ZUSAMMENSETZUNG ZUR SYSTEMISCHEN VERABREICHUNG PHARMAKOLOGISCH AKTIVER WIRKSTOFFE ENTHALTEND MITTELKETTIGE TRIGLYCERIDE**
INJECTABLE PHARMACEUTICAL COMPOSITION FOR SYSTEMIC DELIVERY OF PHARMACOLOGICALLY ACTIVE COMPOUNDS COMPRISING MEDIUM CHAIN TRIGLYCERIDES
COMPOSITION PHARMACEUTIQUE INJECTABLE POUR L'ADMINISTRATION SYSTEMIQUE DE PRINCIPES ACTIFS D'ACTION PHARMACOLOGIQUE COMPRENANT DES TRIGLYCERIDES DE CHAINE MOYENNE

(30) Priorität: 24.08.2000 DE 10041478
(43) Veröffentlichungstag der Anmeldung: 28.05.2003
(73) Patentinhaber: SCHWARZ PHARMA AG, 40789 Monheim (DE)
(72) Erfinder: RIMPLER, Stephan, D-40723 Hilden (DE); GRAPATIN, Sabine, D-40764 Langenfeld (DE); KREIN, Cliff, D-51491 Overath (DE); THELEN, Markus, D-40789 Monheim (DE)
(86) Internationale Anmeldenummer: PCT/EP2001/009596
(87) Internationale Veröffentlichungsnummer: WO 2002/015937

(56) Entgegenhaltungen:
- GB-A- 1 541 710
- GB-A- 2 105 589
- GB-A- 2 232 082
- US-A- 4 722 933

## Beschreibung

Die vorliegende Erfindung betrifft neue pharmazeutische Zusammensetzungen zur systemischen Verabreichung pharmakologisch aktiver Wirkstoffe.

Insbesondere betrifft die Erfindung injizierbare pharmazeutische Zusammensetzung zu Verabreichung eines pharmakologisch aktiver Wirkstoffs über einer Zeitraum von mindestens 24 Stunden umfassend
(a) einen pharmakologisch aktiven Wirkstoff in fester Phase,
(b) ein flüssiges Vehikel, das zu über 90% aus mittelkettigen Triglyceriden mit einem Veresterungsgrad von über 80% besteht
(c) ein Netzmittel, das zu über 90% aus Polyol-Fettsäure-Estern mit einem Mono-ester-Anteil von mindestens 90% besteht.

### Technischer Hintergrund:

Zahlreiche biologisch aktive Substanzen wie bestimmte niedermolekulare pharmazeutische Wirkstoffe, Peptide, Nucleinsäuren, Vaccine oder Hormone werden bevorzugt parenteral verabreicht.

Der Grund ist vielfach ein starker mechanischer, chemischer oder enzymatischer Abbau in Magen, Darm oder Leber von Patienten nach oraler Verabreichung oder aber eine geringe Bioverfügbarkeit wegen unzureichender Resorption aus dem Gastrointestinaltrakt.

Ein Beispiel für einen Wirkstoff mit starkem Abbau ist N-0923 (S(-)-2-(N-propyl-N-2-thienylerhylamino)-5-hyroxytetralin), ein Dopamin D2 Agonist zur Behandlung von Morbus Parkinson. Wegen eines ausgeprägten First-Pass-Effekts liegt die Bioverfügbarkeit nach oraler Dosierung nur bei etwa 0.5 % (Swart and Zeeuw, Pharmazie 47 (1992) 613), so daß orale Verabreichungsformen für N-0923 nicht in Frage kommen.

Weitere Beispiele für Wirkstoffe mit schlechter gastrointestinaler Resorption sind Peptide, Proteine, Enzyme oder Nucleinsäuren, die im allgemeinen oral nicht oder nur zu einer therapeutisch irrelevanten Menge resorbiert werden.

Für diese Wirkstoffe besteht ein großer Bedarf an parenteralen Arzneiformen. Zahlreiche Wirkstoffe haben jedoch auch nach Injektion eine geringe Halbwertzeit, da sie rasch aus dem Körper eliminiert werden.

Auch hierfür ist N-0923 ein Beispiel. Die Halbwertszeit nach Gabe wässriger Lösungen von N-0923 lag in Tierversuchen nach intravenöser Gabe bei 52 Minuten (Walters et al , J Pharmac Sci 83 (1994) 758) und nach subkutaner Gabe bei 60-70 Minuten (Belluzzi, Movement Disorders, 9.2 (1994) 147), was bei einer Dauertherapie zur Notwendigkeit einer für die Patienten inakzeptabel häufigen Verabreichung führen würde.

Insbesondere die Eliminations-Halbwertszeit zahlreicher Peptide und Enzyme ist sehr gering. Beispielsweise hat Insulin nach Injektion in wässriger Lösung eine Halbwertszeit von etwa 6 Minuten und Proinsulin C-Peptid von etwa 30 Minuten.

Ähnliches gilt für die Pharmakokinetik von Nucleinsäuren, Oligonucleotiden oder Nucleosid-Analoga. So hat das in der Krebstherapie eingesetzte 5-Fluorouracil eine Halbwertszeit von nur 10-20 Minuten, ist oral unwirksam und muß in Dauerinfusionen verabreicht werden.

Daher besteht für derartige Substanzen mit niedriger oraler Bioverfügbarkeit und rascher Elimination ein starker Bedarf an systemischen Arzneiformen, durch die die therapeutische Applikationsfrequenz oder Verabreichungsdauer signifikant gesenkt wird.

Eine Methode zur Retardierung injizierter Wirkstoffe ist die Verabreichung in Form von Suspensionen. Bei der Suspendierung von Substanzen in wässrigen Lösungen wird der Wirkstoff beispielsweise mit Metallionen oder geladenen Substanzen ausgefällt, wobei der Wirkstoff reversibel an das lon gebunden wird.

Ein Beispiel hierfür sind wässrige Zink-Insulin oder Zink-Insulin-Protamin-Suspensionen die seit den 30er Jahren bekannt sind. Das Verhältnis von gebundenem zu ungebundenem Wirkstoff ist dabei ein Maß für die zu erwartende Depotwirkung. Ein Beispiel für seine solche Insulin-Zink-Kristallsuspension wird in EP-A-0 025 868 beschrieben.

Die Entwicklung derartiger Depotarzneiformen ist jedoch schwierig und hängt stark von den Charakteristika und individuellen physikochemischen Eigenschaften des jeweiligen Wirkstoffs ab. Die Ergebnisse, die mit einem Wirkstoff wie Insulin gewonnen werden, lassen sich daher nicht oder nur sehr schlecht auf andere Wirkstoffe übertragen.

Ölige Suspensionen wässriger Wirkstoffe sind ebenfalls lange bekannt, haben jedoch in der Regel den Nachteil, dass die Suspensionen so viskos sind, dass sie entweder nicht durch übliche Applikationskanülen gängig oder aber instabil sind, so dass sich schon nach kurzer Lagerung Sedimentkuchen bilden aus denen die Suspensionen nicht mehr vollständig aufgeschüttelt werden können.

Ein Beispiel zur Herstellung stabiler injizierbarer öliger Peptidpräparate wird in der OS DE 2,306,075 vorgeschlagen. Dabei werden die Peptide zunächst mit einem Aluminiumfettsäuresalz gemischt und das resultierende Adsorbat sodann in Öl suspendiert oder aber die Peptide werden in einem öligen Gel, welches ein Fettsäurealuminiumsalz als Gelbildner enthält, suspendiert. Die vorgeschlagene Formulierung hat jedoch den Nachteil, dass sie mit Aluminium ein giftiges Metall enthält, was inbesondere bei wiederholter Verabreichung aus toxikologischer Sicht sehr problematisch ist. Weiterhin ist die Wirkstofffreisetzung aus Gelen schlecht kontrollierbar, die Applikation für den Patienten häufig unangenehm und die Gefahr systemischer Nebenwirkungen bei etwaigen Fehlinjektionen groß.

In ähnlicher Weise offenbart GB 2 232 082 injizierbare thixotrope Gele zur Verabreichung von Annoxicillin enthaltend ein öliges Vehikel in Kombination mit einem Aluminiumfettsäuresalz und einem Netzmittel.

US 5,013,713 offenbart injizierbare Peptidpräparate. Zur Retardierung wird die Überführung der Peptide in schwer lösliche Salze vorgeschlagen, die dann in einem öligen Vehikel suspendiert werden sollen. Bevorzugt sollen weiterhin Dehydrierungsreagenzien wie z.B. Magnesiumstearat oder Fettsäure-Metallsalze zugesetzt werden, die zur Gelbildung führen. Ein Nachteil der vorgeschlagenen Methode ist jedoch, dass die pharmakokinetischen und pharmakologischen Eigenschaften der jeweiligen Metallsalze nur schwer vorhersagbar sind. Zudem neigen die Feststoffpartikel in den vorgeschlagenen pharmazeutischen Formulierungen zur Aggregation und Sedimentation und zur Bildung unlöslicher Sedimentkuchen.

Aufgabe der vorliegenden Erfindung war es daher, eine stabile, aus möglichst wenig Komponenten aufgebaute pharmazeutische Formulierung bereitzustellen, die als injizierbare Depotform für eine Vielzahl von Wirkstoffen geeignet ist.

Die Formulierung sollte die Wirkstoffe über einen Zeitraum von mindestens 24 Stunden kontinuierlich abgeben, und sollte möglichst lagerfähig, gut verträglich, untoxisch, vollständig bioabbaubar und einfach herzustellen sein.

Die Aufgabe wurde erfindungsgemäß gelöst durch die Bereitstellung einer injizierbaren pharmazeutischen Formulierung umfassend einen pharmakologisch aktiven Wirkstoff in fester Phase, ein flüssiges Vehikel, das zur über 90% aus mittelkettigen Triglyceriden mit einem Veresterungsgrad von über 80% besteht, und ein Netzmittel, welches zu über 90% aus Polyol-Fettsäure-Estern besteht, die zu mindestens 90% als Monoester vorliegen.

Überraschenderweise gelingt es mit dieser sehr einfach aufgebauten pharmazeutischen Formulierung einen über 48 Stunden therapeutisch relevanten Wirkstoff-Plasmaspiegel zu erreichen. Dabei ist die erfindungsgemäße Zusammensetzung überraschend einfach und kostengünstig herzustellen, lagerstabil, hitzesterilisierbar und leicht resuspendierbar.

Zudem enthält besagte Zusammensetzung in besonders vorteilhafterweise nur wenige, gut charakterisierte, gut verträgliche und bioabbaubare Hilfsstoffe.

Ein weiterer Vorteil der vorliegenden Erfindung ist die breite Anwendbarkeit. Die erfindungsgemäße pharmazeutische Zubereitung ist für die Verabreichung zahlreicher Wirkstoffe geeignet. So ist es durch individuelle Einstellung des Injektionsvolumens, des Applikationsintervalls und der Wirkstoffkonzentration der erfindungsgemäßen Formulierungen sehr leicht möglich, die Dosierung an die Bedürfnisse, Symptomatik und Verfassung des jeweiligen Patienten anzupassen.

Weiterhin haben die erfindungsgemäßen pharmazeutischen Zubereitungen gegenüber in den Körper verbrachten Langzeitdepots, die zur Verabreichung von Wirkstoffen über 1, 3 oder sogar 6 Monate konzipiert sind, den Vorteil der besseren Steuerbarkeit. So ist es bei manchen Patienten wünschenswert, dass der Wirkstoff in einem überschaubaren Zeitraum wieder aus dem Körper entfernt ist. Dies ist besonders für Therapeutika mit niedriger therapeutischer Breite von Bedeutung.

### Abbildungen

Abbildung 1 zeigt Plasmakonzentrationen von N-0923 nach subkutaner Verabreichung vier verschiedener Dosen N-0923 in öliger Kristallsuspension in der Ratte. Die Verabreichung erfolgte alle 48 Stunden über mehrere Wochen. Abbildung 1A zeigt gemittelte Meßwerte nach der 2. Applikation, Abbildung 1B nach der 46. Applikation.

Abbildung 2 zeigt Plasmakonzentrationen von N-0923 nach subkutaner Verabreichung von 12,5 mg N-0923 pro kg Körpergewicht in der Ratte. Die Verabreichung erfolgte alle 48 Stunden. Wiedergegeben wurden Plasmaspiegel einzelner Tiere jeweils 2, 4, 8, 24, 32 und 48 Stunden nach der 22. Applikation.

Abbildung 3 zeigt Plasmakonzentrationen von N-0923 nach 85maliger Applikation von 1 mg/kg N-0923 (Abb. 3A) und 4 mg/kg N-0923 (Abb. 3B) in Form öliger N-0923 Kristallsuspension im Affen.

Abbildung 4 zeigt die Korrelation zwischen applizierter Dosis N-0923 in Form öliger Kristallsuspensionen und dem maximalen Plasmaspiegel nach 3maliger und 85maliger täglicher Applikation im Affen.

### Beschreibung der Erfindung:

Die vorliegende Erfindung betrifft injibierbare pharmazeutische Zusammensetzungen umfassend
(a) wenigstens einen pharmakologisch aktiven Wirkstoff in fester Phase,
(b) ein flüssiges Vehikel, zu über 90% aus mittelkettigen Triglyceriden mit einem Veresterungsgrad von über 80%, bevorzugt über 90% besteht sowie
(c) ein Netzmittel, das zu über 90% aus Polyol-Fettsäure-Estern besteht, die einen Monoester-Anteil von mindestens 90% aufweisen.
wobei der pharmakologisch aktive Wirksttoff ein Antiparkinsonmittel, ein Antiepileptikum oder ein Mittel zur Behandlung von Neuropathien ist.

Unter dem Ausdruck "feste Phase" wird in dieser Patentanmeldung das Vorliegen des Wirkstoffs in fester Form verstanden. Dabei kann der Wirkstoff in freier fester Form z.B. als Kristalle oder als amorphe Partikel vorliegen oder aber auch an ein geeignetes Trägermaterial gebunden sein. Bevorzugt werden freie Wirkstoff-Kristalle oder amorphe Wirkstoff-Partikel. Besonders bevorzugt werden freie Wirkstoff-Kristalle, in denen die Wirkstoffe als Salze vorliegen.

Unter dem Ausdruck "Vehikel" wird in dieser Patentanmeldung eine bei Raumtemperatur flüssige kontinuierliche Phase verstanden, in der die feste Phase suspendiert vorliegt.

Das Vehikel besteht erfindungsgemäß zu mindestens 90% aus mittelkettigen Triglyceriden mit einem Veresterungsgrad von über 80 %, kann jedoch bis zu 10%, bevorzugt weniger als 5% und besonders bevorzugt weniger als 3% andere Bestandteile, wie z.B. pflanzliche Öle oder Fettsäuren als Beimengungen enthalten.

Unter dem Ausdruck "Netzmittel" wird in dieser Patentanmeldung ein Stoff verstanden, der die Grenzflächenspannung zwischen Vehikel- und Wirkstoffoberfläche herabsetzt.

Das Netzmittel besteht erfindungsgemäß zu mindestens 90% aus Polyol-Fettsäure-Estern, die einen Monoester-Anteil von mindestens 90% aufweisen. Erfindungsgemäß kann das Netzmittel jedoch bis zu 10%, bevorzugt weniger als 5% und besonders bevorzugt weniger als 3% andere Bestandteile, wie z.B. Polyol-Alkohol-Kondensate als Beimengungen enthalten.

Die erfindungsgemäßen pharmazeutischen Zusammensetzungen sind zur parenteralen Verabreichung geeignet.

Insbesondere sind die erfindungsgemäßen Formulierungen zur Verabreichung durch Injektion geeignet, wobei die Injektion sowohl durch herkömmliche Spritzen als auch durch nadellose Injektionssysteme erfolgen kann. Beispiele für solche nadellosen Injektionssysteme sind in den Patentschriften US 5,840,062 und US 4,913,699 beschrieben. Die Injektion kann auf einem der im Stand der Technik bekannten Applikationswege für Depotformen, wie subkutan, intrakutan, intramuskulär oder intrakranial, z.B. intraventrikulär, erfolgen.

Besonders bevorzugt wird die subkutane oder intramuskuläre Verabreichung.

Da das Verhältnis des in der Formulierung gelösten Wirkstoffs zu ungelöstem Wirkstoff sowohl Einluß auf die Retardierungswirkung als auch auf die Stabilität der Formulierung hat, betrifft die Erfindung insbesondere solche Zusammensetzungen, die Wirkstoffe enthalten, die in der pharmazeutischen Zusammensetzung weitgehend unlöslich sind.

Unter dem Ausdruck "in der pharmazeutischen Formulierung weitgehend unlöslich" wird verstanden, dass weniger als 10% des eingesetzten pharmakologisch aktiven Wirkstoffs in der pharmazeutischen Formulierung in gelöster Form vorliegt.

Bevorzugt werden solche pharmazeutischen Formulierungen, in denen der zu verabreichende pharmakologisch aktive Wirkstoff zu weniger als 5%, besonders bevorzugt zu weniger als 3% in gelöster Form vorliegen.

Ein bevorzugter Gegenstand der Erfindung sind daher pharmazeutische Zusammensetzungen, die wasserfrei sind.

Unter dem Ausdruck "wasserfrei" wird hier ein Wasseranteil unter 3%, bevorzugt unter 1% verstanden.

Die Erfindung betrifft weiterhin pharmazeutische Zusammensetzungen, umfassend einen pharmakologisch aktiven Wirkstoff in fester Phase, ein im wesentlichen aus mittelkettigen Triglyceriden aufgebautes Vehikel und ein im wesentlichen aus Polyol-Fettsäure-Monoestern aufgebautes Netzmittel, wobei die pharmazeutische Zusammensetzung ferner dadurch gekennzeichnet ist, das sie eine Depotform darstellt und besagten Wirkstoff über einen Zeitraum von mindestens 24 oder 36 Stunden, bevorzugt über mindestens 48 Stunden in therapeutisch wirksamer Menge freisetzt.

Vorteilhafterweise kann dadurch die Verabreichungsfrequenz des Wirkstoffs auf eine einzelne Applikation pro Tag oder eine Verabreichung alle zwei oder drei Tage gesenkt werden.

Bevorzugt werden weiterhin Ausführungsformen der erfindungsgemäßen pharmazeutischen Formulierungen, deren Wirkstoffgehalt nach spätestens 7 Tagen aus dem in den Körper des Patienten verbrachten Depot entfernt ist. Nach 7 Tagen liegen im Körper vorzugsweise nur noch höchstens 5%, besonders bevorzugt weniger als 1% des Wirkstoffs in Depotform im Körper vor.

Im Gegensatz zu Langzeitdepots, die nach Applikation in Patienten über mehrere Wochen pharmakologisch aktiven Wirkstoff freisetzen, haben die erfindungsgemäßen pharmazeutischen Zusammensetzungen somit den Vorteil der besseren Steuerbarkeit: der Wirkstoff kann bei Bedarf in einem überschaubaren Zeitraum an- und abgeflutet werden.

Die kontinuierliche Phase der erfindungsgemäßen Zusammensetzungen besteht zu über 90% aus mittelkettigen Triglyceriden (MKTs) mit einem Gesamt-Veresterungsgrad von 80-100%, bevorzugt 90-100%.

Durch die Wahl der Kettenlänge und die Zahl der Doppelbindungen der Fettsäure im Vehikel können in besonders einfacher Weise die gewünschten physikochemischen Eigenschaften der Formulierung (z.B. die Viskosität) eingestellt werden.

Bevorzugte Ausführungsformen der Erfindung sind dabei pharmazeutische Zusammensetzungen, in denen das Vehikel zu über 90% aus mittelkettigen Tryglyceriden aufgebaut ist, die Fettsäuren einer Kettenlänge zwischen 6 und 22 C-Atomen, besonders bevorzugt zwischen 6 und 14 C-Atomen und ganz besonders bevorzugt zwischen 8 und 10 C-Atomen enthalten.

Bevorzugt ist das Vehikel dabei aus mittelkettigen Triglyceriden aufgebaut, die zu über 60% gesättigte, besonders bevorzugt zu über 90% gesättigte Fettsäuren enthalten.

Ganz besonders bevorzugt werden mittelkettige Triglyceride (MKTs), die hauptsächlich gesättigte Fettsäuren mit Kettenlängen von 8-10 C-Atomen enthalten und die in Pharmakopöen beschrieben sind.

MKTs sind gut charakterisierte Substanzen, die sich für systemische Verabreichungsformen bewährt haben. Vorteilhafterweise sind MKTs bioabbaubar, nicht reizend und haben exzellente physikochemische Eigenschaften für die Verwendung auch in injizierbaren Arzneiformen. MKTs eignen sich daher ganz besonders als Vehikel für die erfindungsgemäßen pharmazeutischen Zubereitungen.

Beispielhaft genannt sei ein kommerziell erhältlicher Triglycerid-Caprylsäure/Caprinsäureester, der unter dem Handelsnamen Miglyol 812^{R} (Fa. Condea) erhältlich ist.

Der Anteil der kontinuierlichen Phase (des Vehikels) an der pharmazeutischen Zusammensetzung ergibt sich im wesentlichen aus den Konzentrationen von Wirkstoff, Netzmittel und etwaigen übrigen Hilfsstoffen und ist üblicherweise über 70%, bevorzugt 88-99.8%; ganz besonders geeignet ist eine Konzentration von 94-99%.

Als weitere Komponente umfasst die erfindungsgemäße pharmazeutische Zusammensetzung mindestens ein Netzmittel. Die Netzmittel haben die wichtige Aufgabe die Grenzflächenspannung zwischen Vehikel- und Wirkstoffoberfläche herabzusetzen und so die Aggregation der Feststoffpartikel zu vermeiden. Gleichzeitig wird mit dem Zusatz des Netzmittels die Viskosität der pharmazeutischen Zusammensetzung und das Sedimentationsverhalten gesteuert.

Die erfindungsgemäßen pharmazeutischen Zusammensetzungen enthalten als Netzmittel im wesentlichen Polyol-Fettsäure-Ester, deren Monoesteranteil höher als 90% ist.

In einer bevorzugten Ausführungsform besteht das Netzmittel im wesentlichen aus Fettsäure-Estern von Polyolen mit zwei bis sechs C-Atomen, wie z.B. Glycerol, 1,3-Butandiol, 1,3-Propandiol, 1,2,3-Butantriol, 1,2,4-Butantriol, Isopropanol, Saccharose oder Sorbitan.

Besonders bevorzugt werden Fettsäureester von Glycerol oder 1,2,3-Butantriol als Netzmittel eingesetzt.

Die bevorzugte Kettenlänge der Fettsäuren in den Polyol-Fettsäure-Monoestern des Netzmittels ist von 6 bis 22 C-Atomen, besonders bevorzugt von 6 bis 14 C-Atomen.

Besagte Polyol-Fettsäure-Monoester beeinhalten dabei vorzugsweise über 60%, besonders bevorzugt über 90% gesättigte Fettsäuren.

In einer weiteren bevorzugten Ausführungsform der Erfindung besteht das Netzmittel im wesentlichen aus Polyol-Fettsäure-Estern, die mit gesättigten Fettsäuren mit 6-14 C-Atomen verestertes Glycerol oder 1,2,3-Butantriol enthalten.

Ganz besonders bevorzugt werden in Pharmakopoen beschriebene Handelsprodukte verwendet, wie Glycerolmonolaurat, das z.B. unter dem Handelsnamen Imwitor 312^{R} oder Glycerolmonocaprylat, das unter dem Handelsnamen Imwitor 308^{R} (Fa Condea) käuflich erhältlich ist.

Glycerolmonolaurat ist eine gut charakterisierte Substanz, die in Deutschland als Nahrungsmittelzusatz zugelassen ist und die sich als besonders geeignet zur Verwendung in den erfindungsgemäßen Depotformen herausgestellt haben.

In einer ganz besonders bevorzugten Ausführungsform der Erfindung besteht das Netzmittel daher im wesentlichen aus Glycerolmonolaurat und/oder Glycerolcaprylat.

In einer weiteren bevorzugten Ausführungsform ist die pharmazeutische Zusammensetzung frei von Phosphatiden. Überraschenderweise stellte der Erfinder fest, dass der Zusatz des in der Literatur als Netzmittel beschriebenen Lecithins die Retardwirkung der erfindungsgemäßen Zusammensetzung aufhebt. Ein Aspekt der Erfindung ist daher eine pharmazeutische Zusammensetzung, die frei von Lecithin ist.

Der Konzentrationsbereich für das Netzmittel richtet sich nach der Menge des Wirkstoffs. Die Netzmittelkonzentration muß ausreichend sein, um eine Benetzung der Wirkstoffpartikel zu gewährleisten. Dies kann mit geeigneten Tests, die dem Fachmann bekannt sind, in einfacher Weise bestimmt werden. Andererseits muß darauf geachtet werden, dass das gewählte Netzmittel in einer Konzentration eingesetzt wird, in der das Netzmittel noch nicht auskristallisiert.

Ein möglicher Konzentrationsbereich (w/w) für die Netzmittel ist 0.02-10%, bevorzugt 0.1-5%, besonders bevorzugt ist eine Konzentration von 0.5-2,5%, wobei die Konzentration des Netzmittels jeweils der Konzentration des Wirkstoffs sowie den Löslichkeitseigenschaften des Netzmittels angepasst wird.

Die erfindungsgemäße Suspension kann gegebenenfalls weitere Hilfs- und Zusatzstoffe enthalten, die dem Fachmann auf dem Gebiet der Galenik bekannt sind. So ist der Zusatz lipidlöslicher Antioxidanzien, wie z.B. Vitamin E sinnvoll, wenn das Vehikel und/oder das Netzmittel ungesättigte Fettsäuren enthalten. Ferner kann die pharmazeutische Zusammensetzung gegebenenfalls Verdickungsmittel enthalten.

Als pharmakologisch aktive Wirkstoffe kommen Antiparkinsonmittel, Antiepileptika, Mittel zur Behandlung von Neuropathien sowie deren pharmazeutisch akzeptable Salze in Frage, die bei Raumtemperatur in fester Phase vorliegen und die keine oder nur schwache Löslichkeit in aliphatischen Lösungsmitteln, insbesondere in Triglyceriden zeigen und somit in der erfindungsgemäßen pharmazeutischen Formulierung weitgehend unlöslich sind. Beispiele hierfür sind dem Fachmann bekannt und werden in gängigen Pharmakopöen wiedergegeben.

Unter dem Ausdruck pharmazeutisch akzeptable Wirkstoff-Salze" werden Salze verstanden, die die gewünschten pharmakologischen Eigenschaften der Wirkstoffe weitgehend erhalten und keine unerwünschten toxischen Effekte auslösen. Beispiele hierfür sind (a) Additionssalze anorganischer oder organischer Säuren, wie z.B. Hydrogenchlorid, Hydrogenbromid, Phosphorsäure, Essigsäure, Weinsäure, Oxalsäure, Fumarsäure, Apfelsäure, Bernsteinsäure, Zitronensäure sowie Salze deren Anionen (b) Salze mit Metallkationen wie z.B. Natrium, Zink, Calcium, Magnesium, Mangan.

Bevorzugt werden Substanzen oder Substanzklassen mit niedriger oraler Bioverfügbarkeit, z.B. unter 30%, und relativ kurzer Eliminationshalbwertszeit, z.B. unter 3 Stunden. Besonders bevorzugt werden weiterhin Wirkstoff-Kristalle.

Geeignete Wirkstoffe oder Wirkstoffsalze können wasserlöslich sein, können jedoch auch grundsätzlich zum Zwecke der stärkeren Retardierung in schwerlösliche Salze überführt werden. Beispiele sind Pamoat oder Tannat-Salze von Peptiden oder Metallsalzkomplexe niedermolekularer Wirkstoffe.

Beispiele für geeignete Wirkstoffe sind insbesondere
- Anti-Parkinsonmittel, wie N-0923, Levodopa, Methyldopa, Scopolamin-Hydrobromid, Tolterodin-Tartrat oder Propargylaminderivate, deren Metaboliten und Prodrugs dieser Metaboliten, wie in WO 99/03817 und WO 99/48858 beschrieben
- Antiepileptika, wie Phenytoin-Natrium oder Harkoserid
- Mittel zur Behandlung von Neuropathien wie etwa Proinsulin C-Peptid.

Unter dem Ausdruck Proinsulin C-Peptid" wird sowohl das 31 Aminosäuren Verbindungsprotein aus nativem humanem C-Peptid, als auch Fragmente und Varianten davon verstanden, wie sie in der OS WO 98/13384 offenbart sind, sofern diese Fragmente und Varianten zur Behandlung diabetischer Komplikationen, insbesondere zur Behandlung diabetischer Neuropathien geeignet ist.

Die erfindungsgemäße pharmazeutische Zusammensetzung kann einen oder mehrerer solcher Wirkstoffe enthalten. Wesentlich für den Retardierungseffekt ist dabei, dass die zu retardierten Wirkstoffe in fester Phase und im Vehikel weitgehend ungelöst vorliegen.

Ein im Vehikel gelöster Wirkstoff wird hingegen im Körper rasch aus der Formulierung freigesetzt. Durch die Kombination eines Vehikel-unlöslichen Wirkstoffs in fester Phase und eines Wirkstoffs, der im Vehikel gelöst vorliegt, kann somit eine rasche Wirkstoffanflutung mit einer Retardwirkung verbunden werden.

Gerade bei der Behandlung von neurodegenerativen Erkrankungen kann eine parenterale Depot-Applikation besonders vorteilhaft sein, da eine häufige orale Selbstmedikation der Patienten krankheitsbedingt nur schwer sicherzustellen ist. Andererseits sind nichthospitalisierten Patienten längere Infusionen unretardierter Injektabilia mit kurzer Halbwertszeit kaum zuzumuten.

Beim Parkinson Syndrom ist zudem ein häufiges und charakteristisches Symptom der morgendliche Rigor der Patienten, der durch das nächtliche behandlungsfreie Intervall hervorgerufen wird. Daher ist auch aus diesem Grund die Verabreichung von Anti-Parkinson-Mittel durch die erfindungsgemäße Depotformulierung besonders vorteilhaft, da durch die konstante Wirkstofffreisetzung aus den erfindungsgemäßen Depots eine nächtliche Untertherapierung des Patienten vermieden wird.

Ein bevorzugter Wirkstoff zur Verwendung in den erfindungsgemäßen Formulierungen ist Levodopa. Die Halbwertszeit im Plasma ist mit 1- 3 Stunden recht gering. Nach oraler Gabe muß zudem mit beträchtlichem Abbau durch Enzyme der gastrointestinalen Mukosa gerechnet werden. Durch Zugabe von Carbidopa oder anderer MAO-Hemmstoffe kann zwar die Abbaurate von Levodopa durch die Monoaminoxidase (MAO) reduziert werden, dennoch besteht ein großer Bedarf an Formulierungen mit verlängerter Wirkung.

Neueren Erkenntnissen zufolge sollen gleichmäßige Levodopa Plasmaspiegel, wie sie durch Dauerinfusionen hervorgerufen werden, günstigen Einfluß auf die als "on/off-Phänomen" bekannten individuellen Schwankungen der Levodopa-Wirksamkeit sowie auf die Entstehung von Dyskinesien haben, die offenbar durch die schwankenden Plasmaspiegel bei oraler Therpie hervorgerufen werden (Chase, Neurol 44, Suppl 6 (1994) S15).

Besonders bevorzugt wird die erfindungsgemäße Zusammensetzung zur Verabreichung von N-0923-Hydrochlorid. Ganz besonders bevorzugt wird dabei die Formulierung mit kristallinem N-0923 Hydrochlorid. Letzteres kann beispielsweise hergestellt werden, wie in USP 4,564,628 beschrieben. Die Bioverfügbarkeit der erfindungsgemäßen pharmazeutischen Zusammensetzungen beträgt für N-0923 (Rotigotine) etwa 70% und der Wirkstoff wird für über 48 Stunden in therapeutisch wirksamer Menge aus in den Körper verbrachten Depots freigesetzt. Die erreichten Plasmawirkstoffspiegel stehen dabei in linearer Beziehung zur in den Körper verbrachten Dosis.

Geeignete Tagesdosen Rotigotine sind z.B. 0,5-40 mg, bevorzugt 1-20 mg, besonders bevorzugt 2-15 mg und ganz besonders bevorzugt 2-10 mg. Daraus ergeben sich Plasmaspiegel Rotigotine von 0,2-10 ng/ml, bevorzugt 0,3-5 ng/ml und besonders bevorzugt von 0,4-3 ng/ml. Die verabreichte Dosis des jeweiligen Wirkstoffs läßt sich zum einen durch die Konzentration des Wirkstoffs in der Formulierung und zum anderen durch die Wahl des Injektionsvolumens steuern. So kann das Volumen in einem weiten Bereich von 5-2000 µl variiert werden. Bevorzugte Applikationsvolumina sind solche zwischen 10 und 1000 µl, insbesondere zwischen 10 und 500 µl.

Sinnvolle Konzentrationen (w/v) für die pharmakologisch aktiven Wirkstoffe in der erfindungsgemäßen Formulierung ergeben sich in erster Linie aus der therapeutischen Wirksamkeit und der Verträglichkeit des jeweiligen Wirkstoffs. Bevorzugte Konzentrationsbereiche sind 0,01-20%, besonders bevorzugt 0,02-5%, ganz besonders bevorzugt 0.1-2%.

Ein weiterer Aspekt der Erfindung ist eine wasserfreie pharmazeutische Zusammensetzung, umfassend mindestens einen pharmakologisch aktiven Wirkstoff in kristalliner Form, der im wesentlichen unlöslich in der pharmazeutischen Zusammensetzung ist, mindestens ein Mittelkettentriglycerid und mindestens einen Polyol-Fettsäure-Monoester.

Bevorzugte Konzentrationen sind in dieser Ausführungsform 0,02-5% für den Wirkstoff, 0,1-5% für den Glycerolmonoester und 88-99,8% für die Mittelkettentriglyceride. Bevorzugter Glycerol Fettsäure Monoester ist Glycerolmonolaurat.

Schließlich betrifft die Erfindung ein Kit, umfassend eine erfindungsgemäße pharmazeutische Zubereitung und eine Vorrichtung zur Injektion. Bei der Vorrichtung zur Injektion kann es sich um ein noch mit der pharmazeutischen Zusammensetzung zu beschickendes System oder um ein bereits mit der erfindungsgemäßen pharmazeutischen Zubereitung befülltes Injektionssystem handeln. Das Injektionssystem kann mit einer herkömmlichen Kanüle versehen sein oder alternativ auch als nadelloses Injektionssystem zu gebrauchen sein.

Ein Gegenstand der Erfindung ist auch ein Kit, in dem mehrere Dosierungen der erfindungsgemäßen pharmazeutischen Zusammensetzung und mehrere Injektionsvorrichtungen enthalten sind, z.B. ein Wochen- oder Monatsvorrat.

Ein weiterer Aspekt der Erfindung ist ein Kit, umfassend eine pharmazeutische Zusammensetzung zur injektion eines Antiparkinsonmittels und eine orale oder transdermale Verabreichungsform eines Antiparkinsonmittels. Unter "Antiparkinsonmittel" wird dabei jeder Wirkstoff verstanden, der geeignet ist, einen pathologisch veränderten Dopaminstoffwechsel günstig zu beeinflussen und/oder in anderer Weise das Fortschreiten oder Bestehen von Morbus Parkinson therapeutisch oder prophylaktisch zu vermindern oder zu verhindern und/oder die mit Morbus Parkinson verbundenen Begleitsymptome zu lindern.

Beispiele für solche Antiparkinsonmittel sind dem Fachmann bekannt. Den Schutzbereich nicht-limitierende Beispiele für geeignete weitere Wirkstoffe sind Vertreter der Gruppe metabolischer Dopaminvorstufen, Dopaminrezeptoragonisten, Dopamintransportblocker, MAO-Hemmer, Muskarin-Rezeptorantagonisten, Glutamat-Rezeptorantagonisten, Catechol-O-Methyltransferase-Blocker, Neurotrophine, Immunophilin-Liganden, Histamin-Antagonisten, Antioxidanzien, Glutathion Transferase-Aktivatoren, Anti-Apoptose-Wirkstoffe oder Calciumantagonisten.

Geeignete Vertreter sind insbesondere Levodopa, Methyldopa, Biperiden, Paragylin, Rasagilin, Selegilin, Lisurid, Pergolid, Bromocriptin, Cabergolin, Benzatropin, Ropinirol, Amantadin, Memantine, Trihexyphenidyl, Diphenhydramin, Dihydroergocryptin, Tolcapon, Entacapon, Metixen, Procyclidin, Budipin, Bornaprin, Pramipexol, Glial cell line-derived neurotrophic factor (GDNF) und der Brain-derived neurotrophic factor (BDNF).

Ein Kit, das eine injizierbare Antiparkinsonformulierung und eine orale Antiparkinson-Verabreichungsform enthält, kann beispielsweise günstig sein, um ein zu starkes Absinken der Plasmaspiegel bei der Erschöpfung eines Depots und vor dem Applizieren oder Anfluten eines neues Depots zu verhindern oder zu überbrücken.

Eine besonders bevorzugte Ausführungsform ist daher ein Kit aus einer injizierbaren Depotform eines Antiparkinsonmittels und einer oralen, schnell anflutenden Formulierung des gleichen oder eines anderen Antiparkinsonmittels. Beispiele für solche schnell anflutenden oralen Dosierungsformen sind aus dem Stand der Technik bekannt und werden z.B. in der EP A 651 997 beschrieben.

### Ausführungsbeispiele:

### 1. Herstellung und Kristallisierung von N-0923

Die Herstellung und Kristallisierung von kristallinem N-0923 erfolgt wie in USP 4,564,628 beschrieben.

### 2. Herstellung einer N-0923 Suspension enthaltend 1% N-0923 und 1% GML

### (a) Herstellen der kontinuierlichen Phase

1411,2 g Miglyol 812 wurde in eine Duran Flasche eingewogen. 14,4 g Imwitor 312 wurde dem Miglyol zugegeben und im Anschluß für 30 Minuten unter Rühren auf 80°C erwärmt. Die klare Lösung wurde auf Raumtemperatur abgekühlt und gefiltert.

### (b) Herstellen der Suspension

1188 g der unter (b) hergestellten Lösung wurde in einen Glaslaborreaktor überführt, 12 g N-0923 zugesetzt und für 10 Minuten mit einem Ultraturrax bei 10.000 UpM unter Stickstoff homogenisiert. Die Suspension wurde bei laufendem Ultraturrax (2.000 UpM) in Braunglasflaschen abgefüllt.

### 3. Herstellung einer N-0923 Suspension enthaltend 0,5, 1,5 und 2% N-0923 und 0,5%, 1% oder 1,5% GML

Die Herstellung erfolgte wie unter 1 beschrieben, mit entsprechend veränderten Einwaagen.

### 4. Hitzesterilisierung von N-0923

Eine 0,6 %ige wässrige Lösung von N-0923 Hydrochlorid (Ansatz I) und eine 1%ige N-0923 Suspension entsprechend Ausführungsbeispiel 2 (Ansatz II) wurde für 20 Minuten bei 120 °C und 0,2 Pa autoklaviert. Zudem wurde eine 0,5%ige wässrige N-0923 Lösung unter Stickstoffbegasung autoklaviert (Ansatz III). Anschließend wurden photometrisch die Abbaumengen bestimmt.

Beim Autoklavieren der wässrigen Lösungen in den Ansätzen I und III zeigte sich, dass jeweils 1,5 % des N-0923 thermisch in Abbauprodukte zersetzt wird. In Ansatz II wurden dagegen weniger als 0,5% Abbau nachgewiesen.

### 5. Freisetzung von N-0923 aus den erfindungsgemäßen Depots in der Ratte

Sprague-Dawley Ratten erhielten subkutane Bolus-Injektionen öliger N-0923 Kristallsuspension der folgenden Zusammensetzung:

| | |
|---|---|
| N-0923 | 0,5 oder 1 %ig |
| Imwitor 312 | 1 % |
| Miglyol 812 | ad 100 % |

Die Applikation erfolgte alle 48 Stunden in den folgenden Dosierungen:

| | |
|---|---|
| 1 mg/kg | (0,2 ml/kg einer 0,5 %igen Suspension) |
| 3 mg/kg | (0,6 ml/kg einer 0,5 %igen Suspension) |
| 10 mg/kg | (1 ml/kg einer 1 %igen Suspension) |
| 30 mg/kg | (3 ml/kg einer 1 %igen Suspension) |

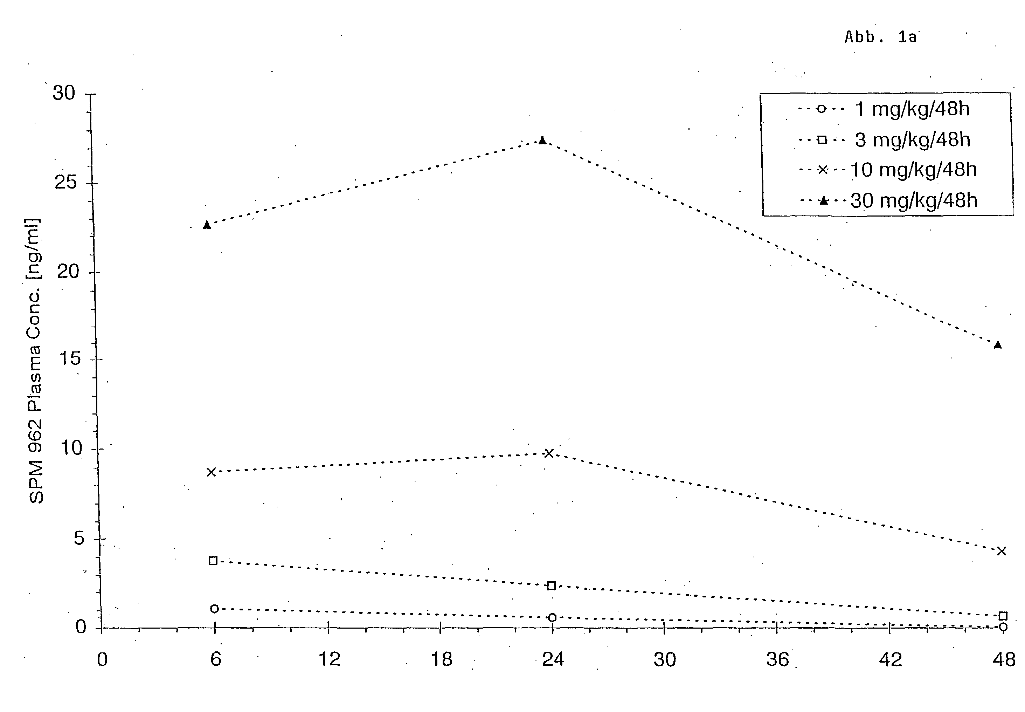

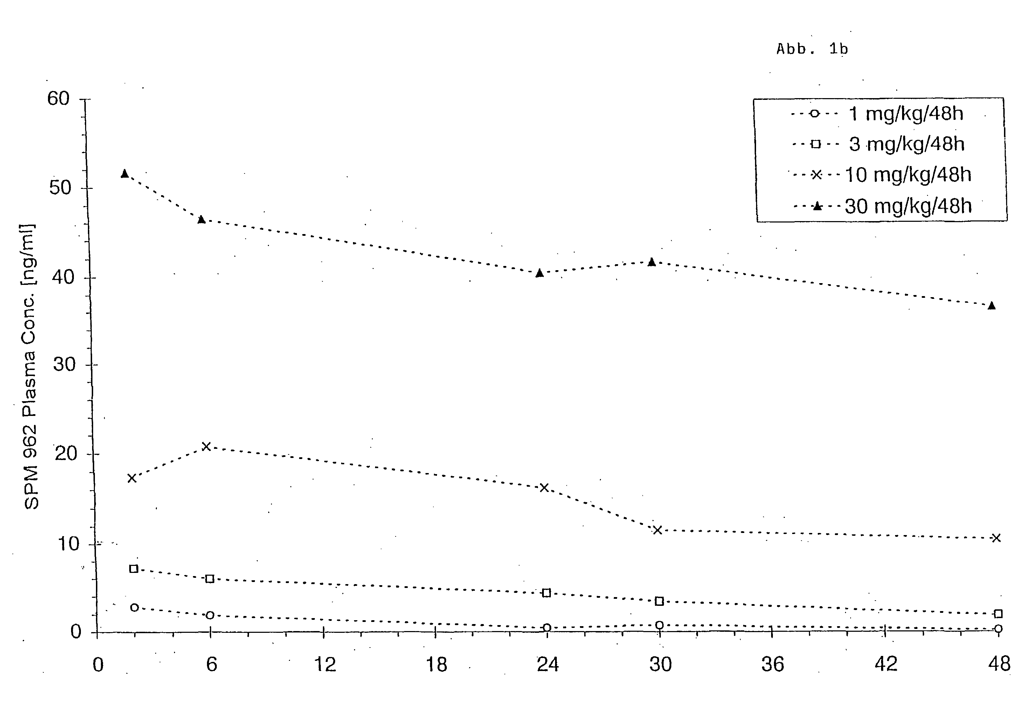

6, 24 und 48 Stunden nach der 2. und 46. Verabreichung wurden Plasmaproben entnommen und die N-0923 Konzentration mittels LC-MS-MS analysiert. Die Werte aus 6 Tieren wurden gemittelt. Die Resultate sind in Abbildung 1 dargestellt.

### 6. Freisetzung von N-0923 aus den erfindungsgemäßen Depots in der Ratte

Die Versuchsanordnung entspricht Ausführungsbeispiel 5, mit dem Unterschied, daß alle 48 Stunden eine Dosis von 12,5 mg N-0923/kg Körpergewicht appliziert wurde.

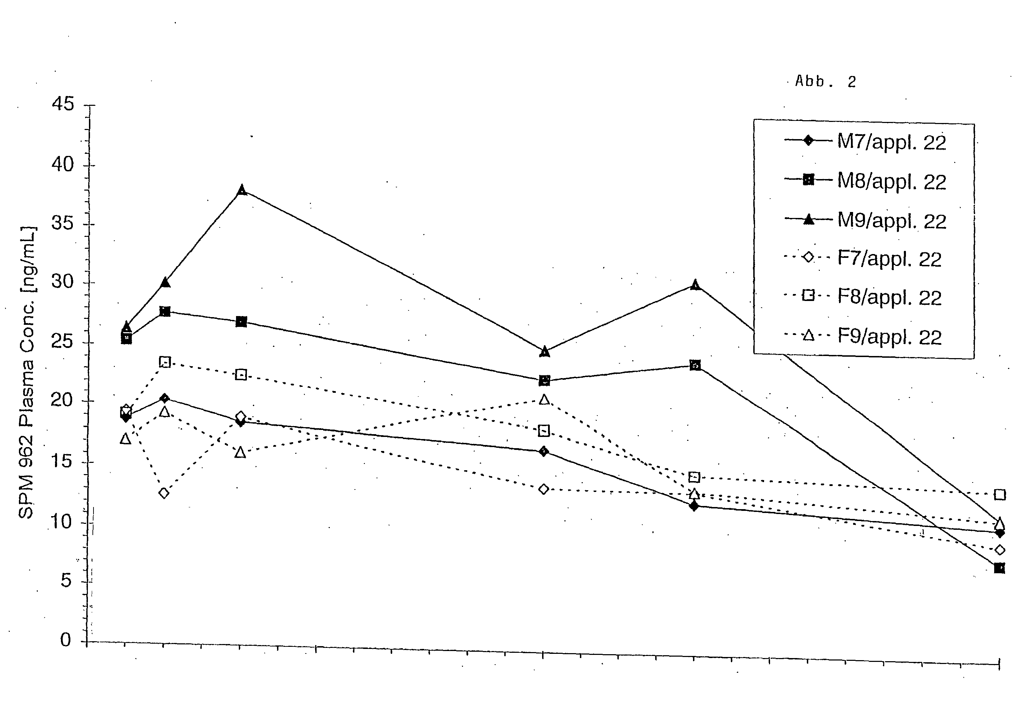

Meßwerte wurden jeweils 2, 4, 8, 24, 32 und 48 Stunden nach der 22. Applikation genommen und quantifiziert. Die Plasmaspiegel der einzelnen Tiere sind in Abbildung 2 dargestellt.

### 7. Freisetzung von N-0923 aus den erfindungsgemäßen Depots im Affen

Cynomolgus-Affen erhielten tägliche subkutane Bolus-Injektionen öliger N-0923 Kristallsuspensionen der folgenden Zusammensetzung:

| | |
|---|---|
| N-0923 | 0,5 oder 1 %ig |
| Imwitor 312 | 1 %ig |
| Miglyol 812 | ad 100 % |

Die Applikation erfolgte täglich in Dosen von 0,25, 1 und 4 mg/kg. 2, 6 und 24 Stunden nach der 3. und 85. Applikation wurden Plasmaproben entnommen und per LC-MS-MS analysiert.

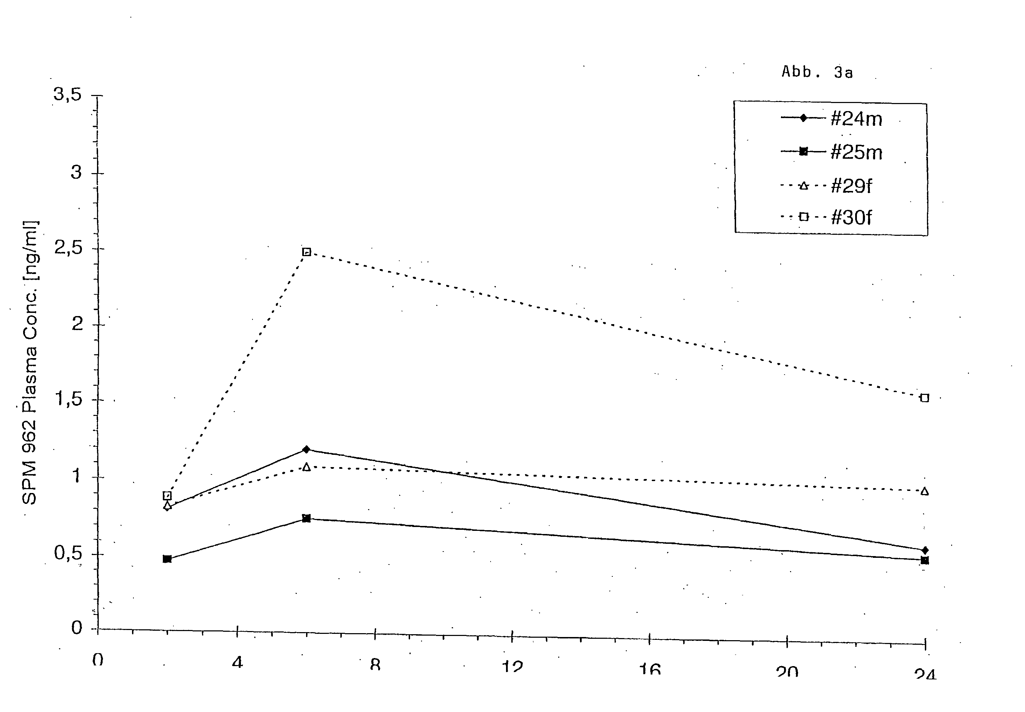

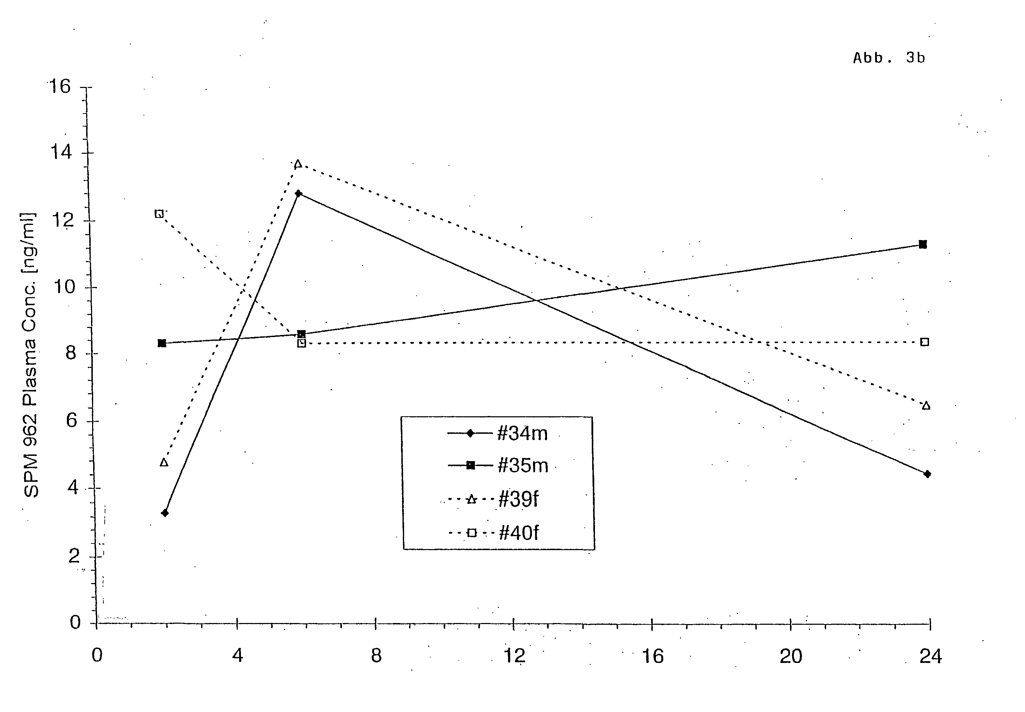

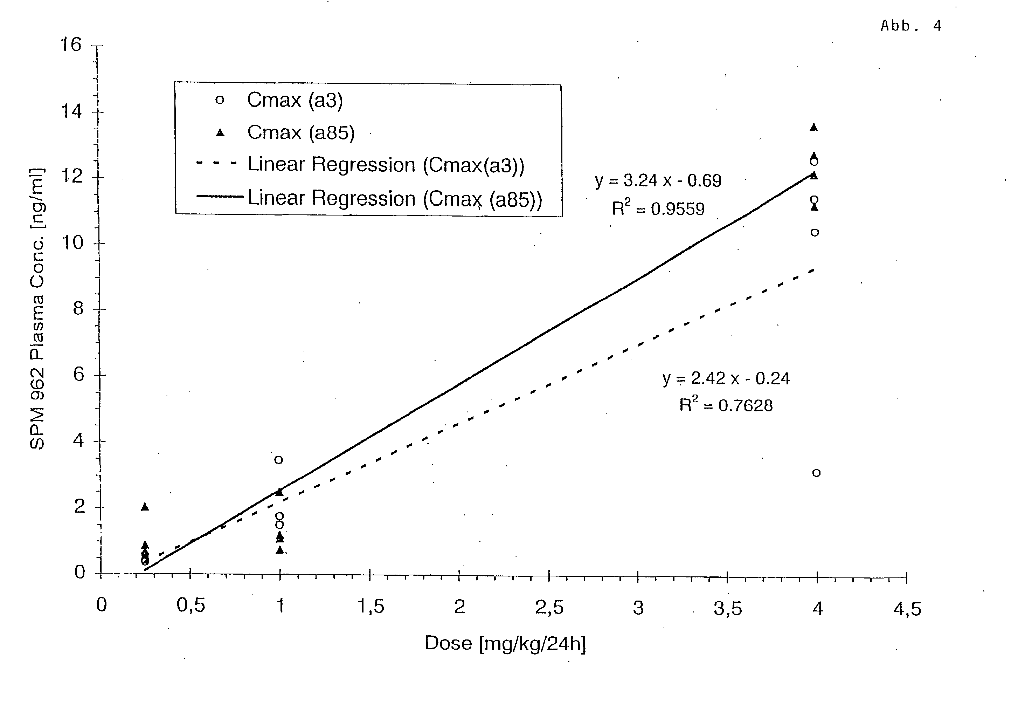

Abbildung 3 zeigt die Meßwerte in einzelnen Tieren. Abbildung 4 zeigt die Beziehung zwischen eingesetzter Dosis und resultierenden maximalen Plasmakonzentrationen

## Patentansprüche

1. Injizierbare pharmazeutische Zusammensetzung zur Verabreichung eines pharmakologisch aktiven Wirkstoffs über einen Zeitraum von mindestens 24 Stunden, **dadurch gekennzeichnet, dass** die injizierbare Zusammensetzung umfasst
(a) wenigstens einen pharmakologisch aktiven Wirkstoff in fester Phase,
(b) ein flüssiges Vehikel, das zu über 90 % aus mittelkettigen Triglyceriden mit einem Veresterungsgrad von über 80% besteht,
(c) ein Netzmittel, das zu über 90 % aus Polyol-Fettsäure-Estern mit einem Monoester-Anteil von mindestens 90 % besteht,
wobei der pharmakologisch aktive Wirkstoff ein Antiparkinsonmittel, ein Antiepileptikum oder ein Mittel zur Behandlung von Neuropathien ist.

2. Pharmazeutische Zusammensetzung nach Anspruch 1, **dadurch gekennzeichnet, daß** die pharmazeutische Zusammensetzung wasserfrei ist.

3. Pharmazeutische Zusammensetzung nach einem der vorhergehenden Ansprüche, **dadurch gekennzeichnet, daß** der pharmakologisch aktive Wirkstoff in der pharmazeutischen Zusammensetzung weitgehend unlöslich ist.

4. Pharmazeutische Zusammensetzung nach einem der vorhergehenden Ansprüche, **dadurch gekennzeichnet, daß** das Netzmittel zu über 90 % aus Polyol-Fettsäure-Estern besteht, die 1,3-Propandiol, Glycerol, 1,2,3-Butantriol, 1,2,4-Butantriol, 1,3-Butandiol, Sorbitan oder Isopropanol als Polyol-Komponente enthalten.

5. Pharmazeutische Zusammensetzung nach einem der vorhergehenden Ansprüche, **dadurch gekennzeichnet, daß** das Netzmittel zu über 90 % aus Polyol-Fettsäure-Estern besteht, die Fettsäurereste mit einer Kettenlänge von 6 bis 14 C-Atomen enthalten.

6. Pharmazeutische Zusammensetzung nach einem der vorhergehenden Ansprüche, **dadurch gekennzeichnet, daß** das Netzmittel zu über 90 % aus Glycerolmonolaurat oder Glycerolmonocaprylat besteht.

7. Pharmazeutische Zusammensetzung nach einem der vorhergehenden Ansprüche, **dadurch gekennzeichnet, daß** das Netzmittel in einer Konz von 0.1-5% (w/w) vorhanden ist.

8. Pharmazeutische Zusammensetzung nach einem der vorhergehenden Ansprüche, **dadurch gekennzeichnet, daß** die pharmazeutische Zusammensetzung frei von Phosphatiden ist.

9. Pharmazeutische Zusammensetzung nach einem der vorhergehenden Ansprüche, **dadurch gekennzeichnet, daß** der pharmakologisch aktive Wirkstoff in kristalliner Form vorliegt.

10. Pharmazeutische Zusammensetzung nach einem der vorhergehenden Ansprüche, **dadurch gekennzeichnet, daß** der pharmakologisch aktive Wirkstoff in amorpher Form vorliegt.

11. Pharmazeutische Zusammensetzung nach einem der vorhergehenden Ansprüche, **dadurch gekennzeichnet, daß** der pharmakologisch aktive Wirkstoff in Salzform vorliegt.

12. Pharmazeutische Zusammensetzung nach einem der vorhergehenden Ansprüche, **dadurch gekennzeichnet, daß** der Wirkstoff in einer Konz von 0.02-5% (w/v) vorliegt.

13. Pharmazeutische Zusammensetzung nach einem der vorhergehenden Ansprüche, **dadurch gekennzeichnet, daß** der Wirkstoff ausgewählt ist aus N-0923 (Rotigotine), Levodopa, Cabergolin, Ropinirol, Pergolid und Pramipexol.

14. Pharmazeutische Zusammensetzung nach einem der vorhergehenden Ansprüche, **dadurch gekennzeichnet, daß** der Wirkstoff Rotigotine ist.

15. Pharmazeutische Zusammensetzung nach einem der vorhergehenden Ansprüche, **dadurch gekennzeichnet, daß** der Wirkstoff Rotigotine-Hydrochlorid ist.

16. Kit umfassend
(a) eine pharmazeutische Zusammensetzung nach einem der vorhergehenden Ansprüche
(b) eine Vorrichtung zur Injektion.

## Claims

1. Injectable pharmaceutical composition for the administration of a pharmacologically active ingredient over a period of at least 24 hours, **characterized in that** the injectable composition comprises
a) at least one pharmacologically active agent in its solid phase,
b) a liquid vehicle consisting for more than 90% of medium-chain triglycerides with an esterification level of over 80%
c) a wetting agent consisting for more than 90% of polyol fatty acid esters with a monoester content of at least 90 %,
whereas the pharmalogically active ingredient is an antiparkinsonian agent, an anti-epileptic or a substance for treating neuropathy.

2. Pharmaceutical composition as in claim 1, **characterized in that** the pharmaceutical composition is anhydrous.

3. Pharmaceutical composition as in one of the preceding claims, **characterized in that** the pharmacologically active agent is largely insoluble in the pharmaceutical composition.

4. Pharmaceutical composition as in one of the preceding claims, **characterized in that** the wetting agent consists for more than 90% of polyol fatty acid esters containing as the polyol component 1,3-propanediol, glycerol, 1,2,3-butanetriol, 1,2,4-butanetriol, 1,3-butanediol, sorbitan or isopropanol.

5. Pharmaceutical composition as in one of the preceding claims, **characterized in that** the wetting agent consists for more than 90% of polyol fatty acid esters containing fatty acid residua with a chain length of 6 to 14 C-atoms.

6. Pharmaceutical composition as in one of the preceding claims, **characterized in that** the wetting agent consists for more than 90% of glycerol monolaurate or glycerol monocaprylate.

7. Pharmaceutical composition as in one of the preceding claims, **characterized in that** the wetting agent is present at a concentration of 0.1-5% (w/w).

8. Pharmaceutical composition as in one of the preceding claims, **characterized in that** the pharmaceutical composition is free of any phosphatides.

9. Pharmaceutical composition as in one of the preceding claims, **characterized in that** the pharmacologically active agent is present in crystalline form.

10. Pharmaceutical composition as in one of the preceding claims, **characterized in that** the pharmacologically active agent is present in amorphous form.

11. Pharmaceutical composition as in one of the preceding claims, **characterized in that** the pharmacologically active agent is present in salt form.

12. Pharmaceutical composition as in one of the preceding claims, **characterized in that** the active agent is present at a concentration of 0.02-5% (w/v).

13. Pharmaceutical composition as in one of the preceding claims, **characterized in that** the active agent is selected from among N-0923 (rotigotine), levodopa, cabergoline, ropinirole, pergolide and pramipexole.

14. Pharmaceutical composition as in one of the preceding claims, **characterized in that** the active agent is rotigotine.

15. Pharmaceutical composition as in one of the preceding claims, **characterized in that** the active agent is rotigotine-hydrochloride.

16. Kit, comprising
a) a pharmaceutical composition per one of the preceding claims
b) an injection device.

## Revendications

1. Composition pharmaceutique injectable destinée à administrer un principe pharmacologiquement actif sur une période d'au moins 24 heures, ***caractérisée en ce que*** la composition injectable comprend :
(a) au moins un principe pharmacologiquement actif en phase solide,
(b) un vecteur liquide qui est composé à plus de 90 % de triglycérides de poids moléculaire moyen présentant un degré d'estérification de plus de 80 %,
(c) un agent de réticulation qui est composé à plus de 90 % d'esters d'acides gras et de polyols avec une proportion de monoester d'au moins 90 %,
le principe pharmacologiquement actif étant un antiparkinsonien, un antiépileptique ou un produit de traitement de neuropathies.

2. Composition pharmaceutique selon la revendication 1, ***caractérisée en ce que*** la composition pharmaceutique est exempte d'eau.

3. Composition pharmaceutique selon l'une quelconque des revendications précédentes, ***caractérisée en ce que*** le principe pharmacologiquement actif est essentiellement insoluble dans la composition pharmaceutique.

4. Composition pharmaceutique selon l'une quelconque des revendications précédentes, ***caractérisée en ce que*** l'agent de réticulation est composé à plus de 90 % d'esters d'acides gras et de polyols qui contiennent du 1,3-propanediol, du glycérol, du 1,2,3-butanetriol, du 1,2,4-butanetriol, du 1,3-butanediol, du sorbitane ou de l'isopropanol comme composant polyol.

5. Composition pharmaceutique selon l'une quelconque des revendications précédentes, ***caractérisée en ce que*** l'agent de réticulation est composé à plus de 90 % d'esters d'acides gras et de polyols qui contiennent des résidus d'acides gras ayant une longueur de chaîne comprise entre 6 et 14 atomes de carbone.

6. Composition pharmaceutique selon l'une quelconque des revendications précédentes, ***caractérisée en ce que*** l'agent de réticulation est composé à plus de 90 % de monolaurate de glycérol ou de monocaprylate de glycérol.

7. Composition pharmaceutique selon l'une quelconque des revendications précédentes, ***caractérisée en ce que*** l'agent de réticulation est présent selon une concentration de 0,1 à 5 % (en poids).

8. Composition pharmaceutique selon l'une quelconque des revendications précédentes, ***caractérisée en ce que*** la composition pharmaceutique est exempte de phosphatides.

9. Composition pharmaceutique selon l'une quelconque des revendications précédentes, ***caractérisée en ce que*** le principe pharmacologiquement actif est présent sous forme cristalline.

10. Composition pharmaceutique selon l'une quelconque des revendications précédentes, ***caractérisée en ce que*** le principe pharmacologiquement actif est présent sous forme amorphe.

11. Composition pharmaceutique selon l'une quelconque des revendications précédentes, ***ca*** ***ractérisée en ce que*** le principe pharmacologiquement actif est présent sous forme de sel.

12. Composition pharmaceutique selon l'une quelconque des revendications précédentes, ***caractérisée en ce que*** le principe actif est présent selon une concentration de 0,02 à 5 % (en poids).

13. Composition pharmaceutique selon l'une quelconque des revendications précédentes, ***caractérisée en ce que*** le principe actif est choisi parmi la N-0923 (rotigotine), la lévodopa, la cabergoline, le ropinirol, le pergolide et le pramipexol.

14. Composition pharmaceutique selon l'une quelconque des revendications précédentes, ***caractérisée en ce que*** le principe actif est la rotigotine.

15. Composition pharmaceutique selon l'une quelconque des revendications précédentes, ***caractérisée en ce que*** le principe actif est le chlorhydrate de rotigotine.

16. Trousse comprenant :
(a) une composition pharmaceutique selon l'une quelconque des revendications précédentes,
(b) un dispositif d'injection.
